(19) Europäisches Patentamt
European Patent Office
Office européen des brevets

(11) Veröffentlichungsnummer: **0 137 193**
**A2**

(12) # EUROPÄISCHE PATENTANMELDUNG

(21) Anmeldenummer: **84109380.0**

(22) Anmeldetag: **08.08.84**

(51) Int. Cl.[4]: **C 07 D 405/04**
A 61 K 31/445

(30) Priorität: **12.08.83 DE 3329186**

(43) Veröffentlichungstag der Anmeldung:
**17.04.85 Patentblatt 85/16**

(84) Benannte Vertragsstaaten:
**AT BE CH DE FR GB IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Bhat, Sujata Vasudev, Dr.**
**B-9, Paramsukh Housing Society, Gavand Path**
**Nawpada Thane 400 602(IN)**

(72) Erfinder: **Shah, Virbala**
**Devkaran Mansion, Vithaldas Road 24**
**Bombay 400 020(IN)**

(72) Erfinder: **Dohadwalla, Alihussein Nomanbhai, Dr.**
**Noman Mansion, Cumballa Hill 139C**
**Bombay 400 036(IN)**

(72) Erfinder: **Mandrekar, Sadashiv Shantaram**
**Darshan Linking Road (Extn.) 8**
**Santa Cruz (West) Bombay 400 054(IN)**

(72) Erfinder: **de Souza, Noel John, Dr.**
**Avanti Central Avenue 702**
**Santa Cruz Bombay 400 054(IN)**

(72) Erfinder: **Dickneite, Gerhard, Dr.**
**Zum Neuen Hieb 31**
**D-3550 Marburg-Cappel(DE)**

(72) Erfinder: **Kurrle, Roland, Dr.**
**Schenkendorfweg 18**
**D-3550 Marburg(DE)**

(72) Erfinder: **Schorlemmer, Hans-Ulrich, Dr.**
**An der Neuen Schule 14**
**D-3556 Weimar 2(DE)**

(72) Erfinder: **Sedlacek, Hans-Harald, Dr.**
**Am Sonnenhang 3**
**D-3550 Marburg(DE)**

(54) **Chromonalkaloid, Verfahren zu seiner Isolierung aus Dysoxylum Binectariferum, und seine Verwendung als Arzneimittel.**

(57) Die vorliegende Erfindung eine Verbindung der Formel

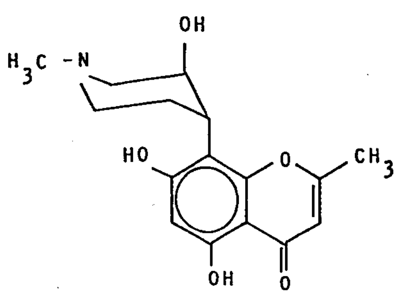

sowie ein Verfahren zu ihrer Isolierung aus Pflanzen der Familie der Meliazeen. Die Verbindung hat insbesondere immunsuppressive Eigenschaften und kann daher als Arzneimittel verwendet werden.

EP 0 137 193 A2

Croydon Printing Company Ltd.

Chromonalkaloid, Verfahren zu seiner Isolierung aus Dysoxylum Binectariferum, und seine Verwendung als Arzneimittel.

Die vorliegende Erfindung betrifft eine pharmakologisch wirksame Substanz, die aus einer zur Familie der Meliazeen gehörigen Pflanze, insbesondere der Pflanze Dysoxylum binectariferum, isoliert werden kann. Die Erfindung betrifft weiterhin ein Verfahren zur Isolierung dieser Substanz aus Dysoxylum binectariferum, gegebenenfalls in Form der freien Base oder ihrer Salze.

Die Familie der Meliazeen umfaßt etwa 550 Pflanzenarten, verteilt auf 50 Gattungen. Von diesen Arten wachsen Dysoxylum, Chisocheton, Sandoricum, Aglaia, Lansium, Amoora, Walsura, Heynea, Beddomea und Xylocarpus in Indien. Die morphologischen Einzelheiten und die Aufteilung dieser Specier sind beschrieben worden in Hooker, "Flora of British India", Bd. I, S. 540-569.

Die erfindungsgemäße pharmakologisch wirksame Substanz wurde aus unterschiedlichen Teilen der Pflanze Dysoxylum binectariferum, d.h. Blättern, Zweigen, Rinde und Holz des Stammes sowie Rinde und Holz der Wurzeln, isoliert.

Gegenstand der Erfindung ist eine pharmakologisch wirksame Substanz mit der Struktur eines Chromonalkaloids der Formel I,

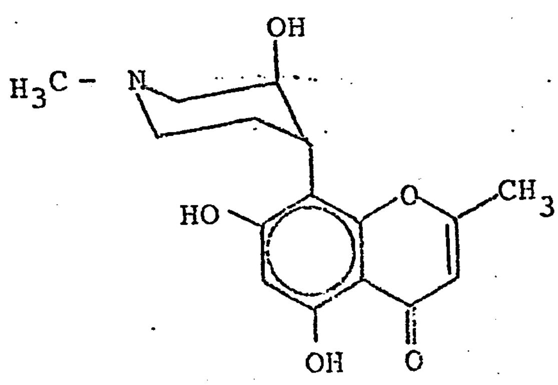

sowie ein Verfahren zur Isolierung dieser Substanz aus Pflanzen der Familie der Meliazeen.

Das Verfahren ist gekennzeichnet dadurch, daß man das getrocknete und gemahlene Pflanzenmaterial zunächst mit einem niederen Alkanol, dann gegebenenfalls mit einem Gemisch Niederalkanol/Alkalihydroxid sowie Niederalkanol/Essigsäure extrahiert, sodann die vereinigten Extrakte zur Gewinnung des pharmakologisch wirksamen Alkaloids wie nachfolgend beschrieben behandelt und gegebenenfalls das Alkaloid in bekannter Weise durch Behandlung mit Säuren in die pharmakologisch verträglichen Salze überführt.

Es ist zweckmäßig, verschiedene Teile der Pflanze Dysoxylum binectariferum wie Blätter, Zweige, Rinde und Holz des Stammes oder der Wurzeln, zu verwenden, insbesondere die Rinde des Stammes in getrockneter und gemahlener Pulverform.

Zur Extraktion der wirksamen Substanz aus Dysoxylum binectariferum kann ein organisches Lösungsmittel wie Methanol, Äthanol, Isopropanol oder ein beliebiger anderer niederer Alkohol eingesetzt werden, wobei Methanol bevorzugt ist. Das Volumen des zur Extraktion eingesetzten Alkanols liegt vorzugsweise im Verhältnis von 1:5 (Gew. Teile Pflanzenmaterial zu Alkanol). Im gleichen Verhältnis wird methanolische Natronlauge (900 ml Methanol + 100 ml NaOH) sowie anschließend methanolische Essigsäure (900 ml Methanol + 100 ml 1 %ige Essigsäure) zur Extraktion verwendet. Die methanolischen Extrakte werden vereinigt, mit Salzsäure auf pH 2 gestellt und mit Chloroform extrahiert. Der Extrakt wird verworfen. Die saure Lösung wird dann mit wäßriger Natronlauge (1 N) auf pH 8 gestellt und die resultierende Lösung gefriergetrocknet. Man erhält so einen festen Rückstand, den man in Methanol löst, filtert und nach Einengen der Methanollösung kristallisieren läßt. Umkristallisieren aus wäßrigem Aceton ergibt ein kristallines Produkt, Fp. 229-232°C, das die erfindungsgemäße pharmakologisch wirksame freie Base darstellt.

Diese freie Base kann dann gegebenenfalls durch Behandlung mit anorganischen und organischen Säuren wie Salzsäure, Nikotinsäure, Maleinsäure und Methansulfonsäure, oder durch Behandlung mit Ammoniumsalzen wie Ammoniumthiocyanat in saure Salze überführt werden.

Die Summenformel der freien Base ist $C_{16}H_{19}NO_5$, berechnet aus dem Molekulargewicht von 305 Masseneinheiten (bestimmt aus dem Massenspektrum) sowie aus der Elementaranalyse. Das UV-Spektrum in Methanol zeigt Absorption bei $\lambda_{max}$ 210, 228, 266 und 326 nm. Das IR-Spektrum wurde an KBr-Pellets gemessen und das NMR-Spektrum mit deuteriertem Pyridin. Fig. 1 und 2 zeigen jeweils das IR- und das NMR-Spektrum.

Die in Methanol gemessene spezifische Drehung $[\alpha]_D^{25}$ hat den Wert +44.31 (c: 13 mg/ml in Methanol).

Die verschiedenen aus der freien Base hergestellten Säureadditionssalze sind im folgenden aufgeführt:

| Salz | Molekularformel | Fp. °C |
|---|---|---|
| Hydrochlorid | $C_{16}H_{20}ClNO_5 \cdot 1/2\ H_2O$ | 228-243 |
| Nicotinat | $C_{22}H_{24}N_2O_7 \cdot 1/2\ H_2O$ | 188-190 |
| Maleinat | $C_{20}H_{23}NO_9$ | 186-188 |
| Methansulfonat | $C_{17}H_{23}NO_8S \cdot 1/2\ H_2O$ | 222-224 |
| Thiocyanat | $C_{17}H_{20}N_2O_5S$ | 224-228 |

Die pharmakologisch wirksame freie Base zeigt chemische und physikalische Eigenschaften, wie sie bei Chromonen und Alkaloiden üblich sind. Alle physikalischen und chemischen Daten bestätigen die der freien Base zugeschriebenen Chromonalkaloid-Struktur. In der Literatur (A.D. Harmon, V. Weiß und J.V. Silverton, Tetrahedron Letters, 721 (1979)) wird eine Verbindung namens Rohitukine

mit der gleichen Summenformel genannt, die aus der Pflanze Amoora rohituka isoliert worden sein soll. Die bisher vorliegenden Angaben reichen nicht aus, um festzustellen, ob stereochemische Unterschiede zwischen Rohitukine und der erfindungsgemäßen Verbindung bestehen.

Das obige pharmakologisch wirksame Chromonalkaloid und seine Säureadditionssalze zeichnen sich durch sehr gute entzündungshemmende, analgetische und immunmodulatorische Eigenschaften bei Labortieren und in in vitro-Versuchen aus.

Das erfindungsgemäße Chromonalkaloid kann insbesondere eingesetzt werden bei der Behandlung von unerwünschten Reaktoren des Immunsystems, gegeben bei Autoimmunerkrankungen, die in der Regel durch Antikörper bedingt sind, hyperergischen oder allergischen Zuständen des Organismus sowie bei chronischen Entzündungsreaktionen, an denen hauptsächlich Makrophagen und Granulozyten beteiligt sind. Ferner kann das Chromonalkaloid verwendet werden als immunsuppresives Mittel zur Verhinderung der Abstoßungsreaktionen von Organtransplantaten, bei der lymphozyten und Makrophagen eine wesentliche Rolle spielt.

Die folgenden Beispiele sollen die Erfindung näher erläutern.

Beispiel 1:

Die getrocknete und gemahlene Rinde des Stammes von Dysoxylum binectariferum (24 kg) wird dreimal mit je 50 l Methanol, dann dreimal mit je 50 l Methanol/Natronlauge (900 ml Methanol + 100 ml 0.1 N NaOH), anschließend dreimal mit je 50 l Methanol/Essigsäure (900 ml Methanol + 100 ml 1%ig Essigsäure) extrahiert. Die vereinigten Methanolextrakte werden mit Salzsäure auf pH 2 gestellt, und die Lösung wird mit Chloroform extrahiert. Der Chloroformextrakt wird verworfen, und die saure Lösung mit wäßriger Natronlauge (1 N) auf pH 8 gestellt. Die basische Lösung wird dann gefriergetrocknet und ergibt einen festen Rückstand (475 g), der mit 7 l Methanol behandelt und gefiltert wird. Das Filtrat wird eingeengt und zur Kristallisation gebracht. Die erhaltenen Kristalle werden gefiltert (415 g) und aus wäßrigem Aceton umkristallisiert. Man erhält so das pharmakologisch wirksame Alkaloid gemäß der Erfindung (300 g) Fp. 229-232°C.

Beispiel 2

In gleicher Weise wie in Beispiel 1, jedoch unter Einsatz von getrockneter und gemahlener Wurzelrinde anstatt der Rinde des Stamms, wurde die Substanz vom Schmp. 229-232°C erhalten.

Beispiel 3 (Nikotinat-Salz)

Die wirksame Base aus Beispiel 1 wurde in Methanol gelöst, mit einer äquivalenten Menge Nikotinsäurelösung in Methanol behandelt, und die Mischung wurde zur Trockne eingedampft. Man erhielt einen festen Rückstand, der, umkristallisiert aus Methanol/Essigester, das Nikotinat-Salz, Fp. 188-190°C, ergab.

Beispiel 4 (Maleinat-Salz)

Analog der Arbeitsweise von Beispiel 3, jedoch unter Einsatz von Maleinsäure anstelle von Nikotinsäure, wurde das Maleinatsalz, Fp. 186-188°C erhalten (Methanol/Essigester).

Beispiel 5 (Methansulfonat-Salz)

Analog der Arbeitsweise von Beispiel 3, jedoch unter Einsatz von Methansulfonsäure anstelle von Nikotinsäure, wurde

0137193

das Methansulfonatsalz, Fp. 222-224°C (Methanol/Essigester) erhalten.

Beispiel 6 (Hydrochlorid-Salz)

Analog der Arbeitsweise von Beispiel 3, jedoch unter Einsatz von Salzsäure anstelle von Nikotinsäure, wurde das Hydrochlorid, Fp. 228-234°C (Wasser/Aceton) erhalten.

Beispiel 7 (Thiocyanat-Salz)

Analog der Arbeitsweise von Beispiel 3, jedoch unter Einsatz von Ammoniumthiocyanat anstelle von Nikotinsäure, wurde das Thiocyanat, Fp. 224-228°C (Methanol/Essigester) erhalten.

Die Modelle zum Nachweis der immunsuppressiven Potenz der erfindungsgemäßen Substanz (im folgenden kurz DBCA genannt) umfaßten Tests zur Erfassung der Wirkung auf Lymphozyten, Makrophagen und Granulozyten in vitro sowie der humoralen Immunität, gemessen als Antikörperantwort gegen eine Palette von Antigenen (thymusabhängige und thymusunabhängige Antigene) in vivo.

Die hier dargestellten Modelle wurden speziell ausgewählt, um die immunsuppresive Potenz dieser Substanz zu charakterisieren.

Wirkung von DBCA auf die humorale Immunantwort

Test 1: Wirkung auf die Antikörperantwort gegen Schafserythrozyten in vivo (thymusabhängiges Antigen).

Weibliche NMRI-Mäuse wurden mit $10^6$ Schafserythrozyten intravenös immunisiert. Gleichzeitig wurde DBCA in den Konzentrationen 5, 10, 20, oder 50 mg/kg intravenös appliziert. Die Kontrollgruppe erhielt das Lösungsmittel

physiologisch gepufferte Kochsalzlösung (PBS). Nach sechs Tagen wurde der Serumspiegel an lysierenden bzw. agglutinierenden Antikörpern gegen Schafserythrozyten bestimmt. Wie Tab. 1 zeigt, wurde der Titer an lysierenden und an agglutinierenden Antikörpern durch die erfindungsgemäße Substanz erniedrigt.

Tabelle 1

Wirkung von DBCA auf die Antikörperantwort gegen Schafserythrozyten in vivo

| | Agglutinierende Antikörper | Lysierende Antikörper |
|---|---|---|
| Kontrolle | 7.6 ± 0.9 | 6.6 ± 0.9 |
| 5 mg/kg i.v. | 5.8 ± 1.3 | 4.0 ± 1.6 |
| 10 mg/kg i.v. | 5.6 ± 0.5 | 4.6 ± 1.3 |
| 20 mg/kg i.v. | 5.4 ± 0.5 | 3.6 ± 0.5 |
| 50 mg/kg i.v. | 4.6 ± 1.3 | 3.0 ± 1.2 |

Test 2: Wirkung auf die IgM-Antikörperantwort gegen E. coli-Totkeime in vivo (thymusunabhängiges Antigen).

Weibliche Mäuse wurden intravenös mit $10^8$ hitzeinaktivierten E. coli-Bakterien immunisiert. DBCA wurde wie in Beispiel 1 angeführt injiziert. 10 Tage nach der Immunisierung wurden die Antikörper der IgM-Klasse gegen E. coli mit Hilfe der ELISA-Technik bestimmt. Wie Abb. 2 zeigt, nahm die optische Dichte ($E_{492nm}$), die ein Maß für die Antikörperkonzentration ist, durch die Gabe von DBCA stark ab.

Tabelle 2

Wirkung von DBCA auf die Antikörperantwort gegen E. coli Totkeime

| | $E_{492nm}$ (1:40) |
|---|---|
| Kontrolle | 1,528 ± 0,332 |
| 5 mg/kg i.v. | 0,402 ± 0,091 |
| 10 mg/kg i.v. | 0,262 ± 0,100 |
| 20 mg/kg i.v. | 0,228 ± 0,052 |
| 50 mg/kg i.v. | 0,179 ± 0,024 |

Test 3: Wirkung von DBCA auf die Antikörperantwort gegen Human-Serum-Albumin (HSA) in vivo

Weibliche Mäuse wurden intravenös mit 100 µg HSA immunisiert. DBCA wurde wie im Beispiel 1 angeführt, injiziert. 14 Tage später wurden die Antikörper der IgG-Klasse gegen HSA mit Hilfe der ELISA-Technik bestimmt. Die Tabelle 3 zeigt, daß die Antikörperantwort durch die erfindungsgemäße Substanz supprimiert wurde.

Tabelle 3

Wirkung von DBCA auf die Immunantwort gegen HSA

| | $E_{492nm}$(1:10) |
|---|---|
| Kontrolle | 0,336 ± 0,215 |
| 10 mg/kg i.v. | 0,194 ± 0,169 |
| 20 mg/kg i.v. | 0,126 ± 0,082 |
| 50 mg/kg i.v. | 0,138 ± 0,081 |
| 100 mg/kg i.v. | 0,175 ± 0,175 |

Test: Wirkung auf die Antikörperantwort gegen Tetanus-Toxoid in vivo

Weibliche NMRI-Mäuse wurden intravenös mit 300 Lf Tetanus-Toxoid immunisiert. DBCA wurden wie in Beispiel 1 angegeben, injiziert. 10 Tage später wurden die Antikörper der IgG-Klasse gegen Tetanus-Toxoid mit Hifle der ELISA-Technik bestimmt. Tabelle 4 zeigt die Reduktion der Antikörperantwort durch DBCA.

Tabelle 4

Wirkung von DBCA auf die Antikörperantwort gegen Tetanus Toxoid

| | $E_{492nm}$ (1:10) |
|---|---|
| Kontrolle | 0,253 ± 0,163 |
| 5 mg/kg i.v. | 0,192 ± 0,168 |
| 10 mg/kg i.v. | 0,150 ± 0,068 |
| 20 mg/kg i.v. | 0,195 ± 0,046 |
| 50 mg/kg i.v. | 0,112 ± 0,026 |

Test 5: Wirkung auf die Antikörper-produzierenden Milzzellen in vitro.

Weibliche NMRI-Mäuse wurden mit $10^8$ Schafserythrozyten intravenös immunisiert. Nach 10 Tagen wurden die Milzen entnommen die vereinzelten Milzzellen wurden in Costar-Platten (5-8x$10^6$ Zellen pro Vertiefung) kultiviert. DBCA wurde in Konzentrationen von 0.1-10 µg/ml den Kulturen zugesetzt. Nach drei Tagen wurden die antikörperbildenden Milzlymphozyten (IgG, IgM) entsprechend dem Plaque forming cell-Test nach Jerne bestimmt. Tabelle 5 zeigt, daß sowohl die IgG als auch die IgM sezernierenden Milzlymphozyten supprimiert wurden.

Tabelle 5

Wirkung von DBCA auf die Antikörper-sezernierenden Milzlymphozyten

| | Plaque forming cells / $10^6$ Milzlymphozyten | | | | | |
|---|---|---|---|---|---|---|
| | IgG | | | IgM | | |
| Kontrolle | 1949 | ± | 971 | 1716 | ± | 870 |
| 0,1 µg/ml | 1665 | ± | 1056 | 1323 | ± | 164 |
| 0,5 µg/ml | 842 | ± | 281 | 1912 | ± | 1041 |
| 0,75 µg/ml | 367 | ± | 162 | 859 | ± | 396 |
| 1,0 µg/ml | 197 | ± | 166 | 1128 | ± | 452 |
| 2,5 µg/ml | ∅ | | ∅ | 289 | ± | 68 |
| 5,0 µg/ml | ∅ | | ∅ | ∅ | | ∅ |
| 10,0 µg/ml | ∅ | | ∅ | ∅ | | ∅ |

Test 6: Einfluß von DBCA auf die Stimulation muriner und humaner Phagozyten.

Es wurden mononukleäre Phagozyten und neutrophile Granulozyten aus peripherem Blut gesunder Spender und Maus-Peritonealmakrophagen gewonnen und nach Gabe des Präparates auf verschiedene Funktionen geprüft. Als Parameter der Phagozytenfunktion wurden die Chemolumineszenzreaktion und die Sekretion lysosomaler Enzyme untersucht.

Verglichen mit Phagozyten der entsprechenden Kontrollgruppen (unbehandelte Zellen) war die Chemolumineszenzreaktion sowohl bei Mausmakrophagen als auch bei humanen Phagozyten (Monozyten und Granulozyten) dosisabhängig (40-400 µg/ml) signifikant erniedrigt (siehe Tabelle 6a). Diese supprimierende Wirkung von DBCA ist noch deutlicher bei einer Co-Stimulation mit Zymosan (100 µg/ml) ausgeprägt.

Maus-Peritonealmakrophagen, die mit der Substanz behandelt werden, zeichnen sich auch besonders dadurch aus,

daß bei ihnen die Sekretion lysosomaler Enzyme deutlich inhibiert ist (Tabelle 6b).

Tabelle 6 a

Oxidativer Stoffwechsel (Chemilumineszenz) mit und ohne Zymosan (100 µg/ml)

| Zelltyp | DBCA | Integral der RLU/15 min ($x10^3$) | |
|---|---|---|---|
| | µg/ml | - Zymosan | + Zymosan |
| Maus- | 0 | 1185 ± 49 | 4686 ± 275 |
| Makrophagen | 50 | 1097 ± 23 | 3835 ± 195 |
| | 100 | 874 ± 31 | 2047 ± 147 |
| | 200 | 523 ± 46 | 1175 ± 35 |
| | 400 | 345 ± 38 | 571 ± 29 |
| Human- | 0 | 2130 ± 71 | 6365 ± 189 |
| Monozyten | 50 | 1905 ± 149 | 5832 ± 162 |
| | 100 | 1511 ± 32 | 4221 ± 157 |
| | 200 | 955 ± 78 | 2668 ± 74 |
| | 400 | 434 ± 43 | 1235 ± 49 |
| Human- | 0 | 3142 ± 127 | 9389 ± 217 |
| Granulozyten | 50 | 2897 ± 276 | 7551 ± 458 |
| | 100 | 1927 ± 152 | 5004 ± 191 |
| | 200 | 864 ± 66 | 1997 ± 107 |
| | 400 | 248 ± 15 | 542 ± 38 |

0137193

Tabelle 6 b

Einfluß der Prüfsubstanz auf die Enzymfreisetzung lysosomaler Hydrolasen von Maus-Peritoneal-Makrophagen.

| Substanz µg/ml | ß-Glu mU/ml | ß-Gal mU/ml | ß-Ac-Glu mU/ml |
|---|---|---|---|
| 0 | 1956 ± 108 | 16295 ± 327 | 5521 ± 264 |
| 50 | 1793 ± 125 | 13596 ± 272 | 4676 ± 182 |
| 100 | 1197 ± 69 | 9058 ± 148 | 2872 ± 159 |
| 200 | 736 ± 23 | 4584 ± 186 | 1749 ± 65 |
| 400 | 348 ± 30 | 1731 ± 102 | 1128 ± 38 |

Test 7: Einfluß von DBCA auf die Mitogen-induzierte Lymphozytenproliferation (human).

Phytohemagglutinin (PHA), ein T-Zellmitogen und Pokeweed-Mitogen (PWM), ein T-Zell-abhängiges B-Zellmitogen, stimulieren bekanntermaßen humane periphere Lymphozyten zu einer polyclonalen Differenzierung und Proliferation. Die Effektivität der Lymphozytenaktivierung wird über den Einbau von $^{14}C$-Thymidin gemessen. Zu Human-Lymphozyten, die in einem derartigen Proliferationstest mit den Mitogenen PHA bzw. PWM stimuliert wurden, wurde an Tag 0 (Kulturbeginn) DBCA in unterschiedlichen Konzentrationen (0.1 - 500 µg/ml) zugegeben und nach 3 Tagen der $^{14}C$-Thymidineinbau gemessen. Die in Fig. 3 exemplarisch dargestellten Ergebnisse zeigen, daß Spender-abhängig DBCA-Konzentrtionen von > 4 µg/ml sowohl die PHA-induzierte als auch die PWM-induzierte Lymphozytentransformation komplett inhibieren. Dieser Effekt ist nicht auf eine unspezifische Toxizität zurückführbar, da DBCA in einer Konzentration von 1 mg/ml (24 Std. Inkubation) nicht toxisch für Humanlymphozyten ist (Vitalfärbung).

Weitere Proliferationsexperimente zeigten, daß DBCA auch nach erfolgter Lymphozytenaktivierung inhibitorisch wirksam sein kann.

Test 8: Einfluß von DBCA auf die PWM-induzierte in vitro IgM-Synthese (human).

Die Aktivierung der zellulären Komponenten des Immunsystems bei einer Immunreaktion läßt sich prinzipiell in die Stufen Induktion, Proliferation, Differenzierung und Effektorleistung gliedern. Um zu prüfen, ob DBCA außer in der Induktions- und Proliferationsphase (siehe Beispiel 7) auch suppresive inhibitorische Auswirkungen auf die Effektorphase einer Immunreaktion besitzt, wurde DBCA in unterschiedlichen Konzentrationen (0.1 - 500 µg/ml) zu PWM-stimulierten Lymphozytenkulturen zugegeben.

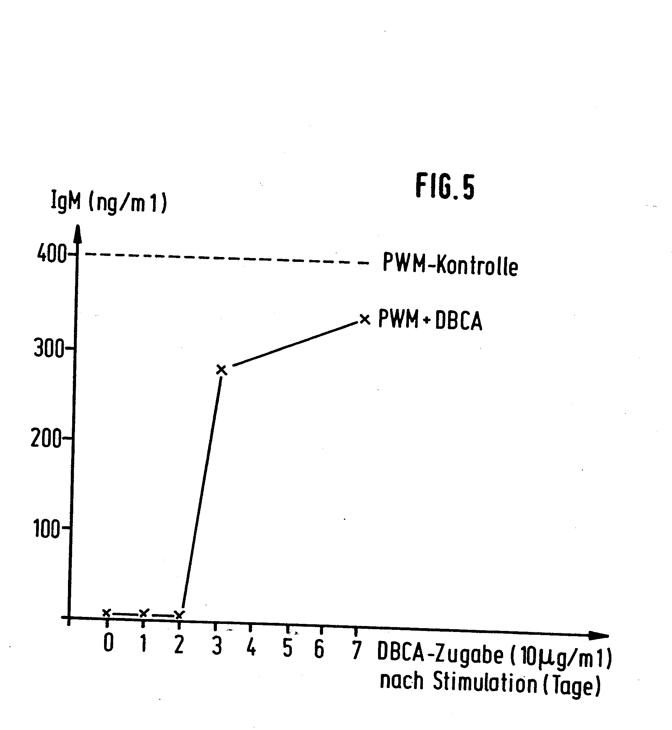

Die in Fig. 4 dargestellten Ergebnisse zeigen, daß in Abhängigkeit vom Lymphozytenspender, DBCA-Konzentrationen von > 10 µg/ml die in vitro-induzierte IgM-Synthese komplett supprimieren. Wird DBCA in einer Konzentration von > 10 µg/ml an unterschiedlichen Tagen nach Induktion der IgM-Synthese (Messung der IgM-Menge an Tag 9) einem derartigen Kultursystem zugesetzt, so zeigt sich Fig. 5, daß DBCA suppressiv auf die Induktions- bzw. Proliferationsphase der Lymphozytenaktivierung wirkt.

PATENTANSPRÜCHE:

1. Verbindung der Formel I

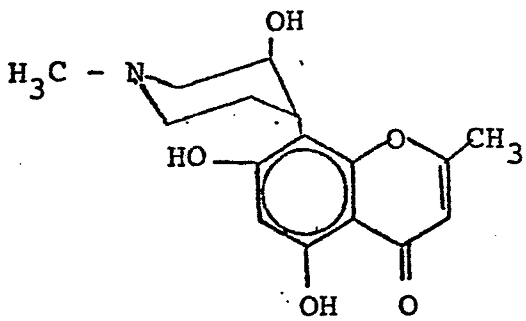

(I)

sowie deren physiologisch verträgliche Säureadditionssalze.

2. Verfahren zur Herstellung einer Verbindung der Formel I gemäß Anspruch 1, dadurch gekennzeichnet, daß man sie aus der Pflanze Dysoxylum binectariferum isoliert.

3. Verfahren zur Isolierung einer Verbindung der Formel I gemäß Anspruch 2, dadurch gekennzeichnet, daß man die Pflanze Dysoxylum binectariferum zunächst mit einem niederen Alkanol, gegebenenfalls anschließend mit einer Mischung aus einem niederen Alkanol und Alkalilauge und schließlich einer Mischung aus einem niederen Alkanol und Essigsäure extrahiert, die alkoholischen Extrakte vereinigt, nach Ansäuern und Extraktion mit Chloroform die verbleibende saure Lösung schwach alkalisch macht und die resultierende Lösung gefriertrocknet; und die feste Substanz umkristallisiert.

4. Arzneimittel, gekennzeichnet durch einen Gehalt an der Verbindung der Formel I oder einem ihrer pysiologisch verträglichen Säureadditionssalze gemäß Anspruch 1.

5. Verbindung der Formel I oder ein physiologisch verträgliches Säureadditionssalz gemäß Anspruch 1 zur Verwendung als Immunsuppressivum.

0137193

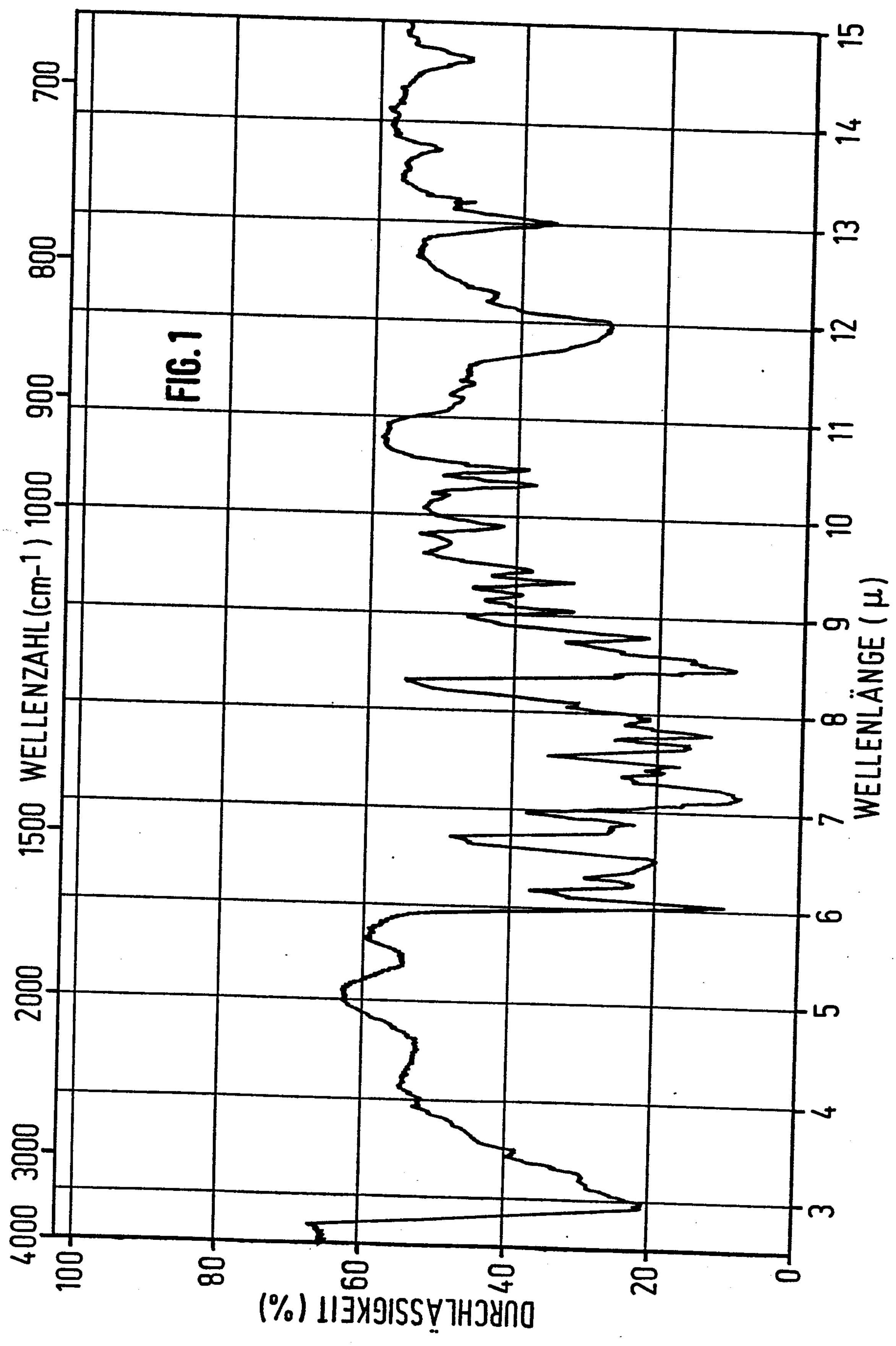


FIG. 1

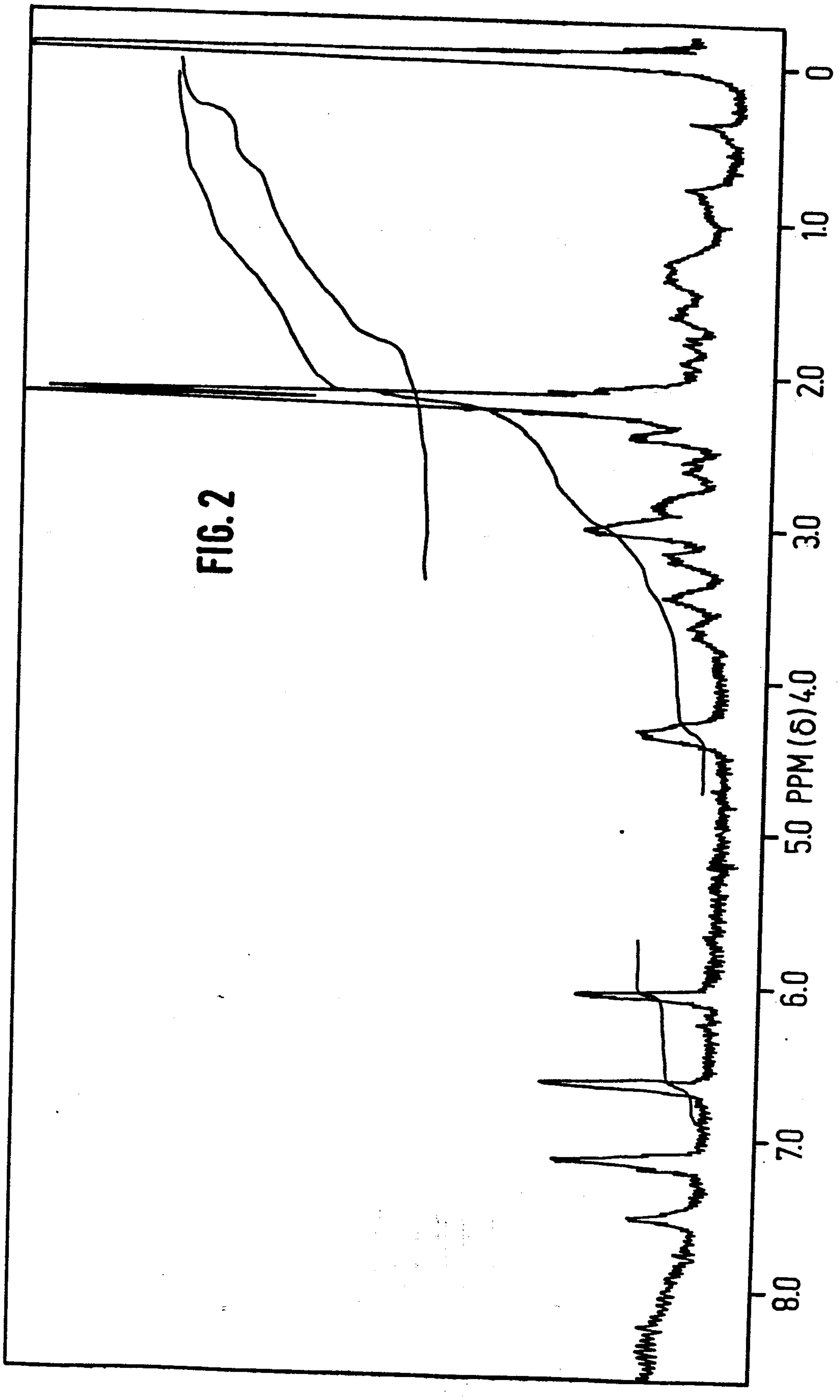


FIG. 2

FIG. 3

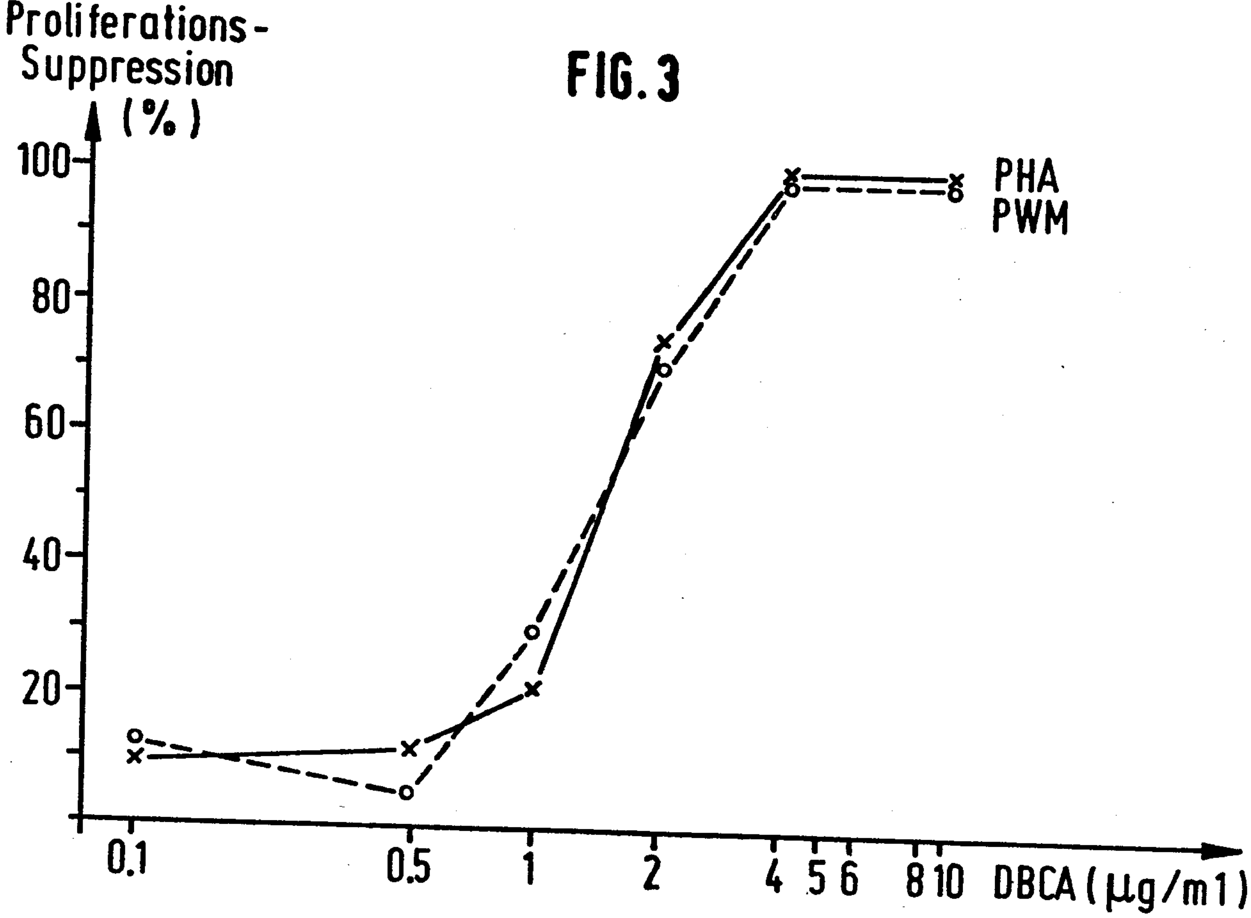
Proliferations-
Suppression
(%)
100
80
60
40
20
PHA
PWM
0.1
0.5
1
2
4
5
6
8
10
DBCA (μg/ml)

FIG. 4

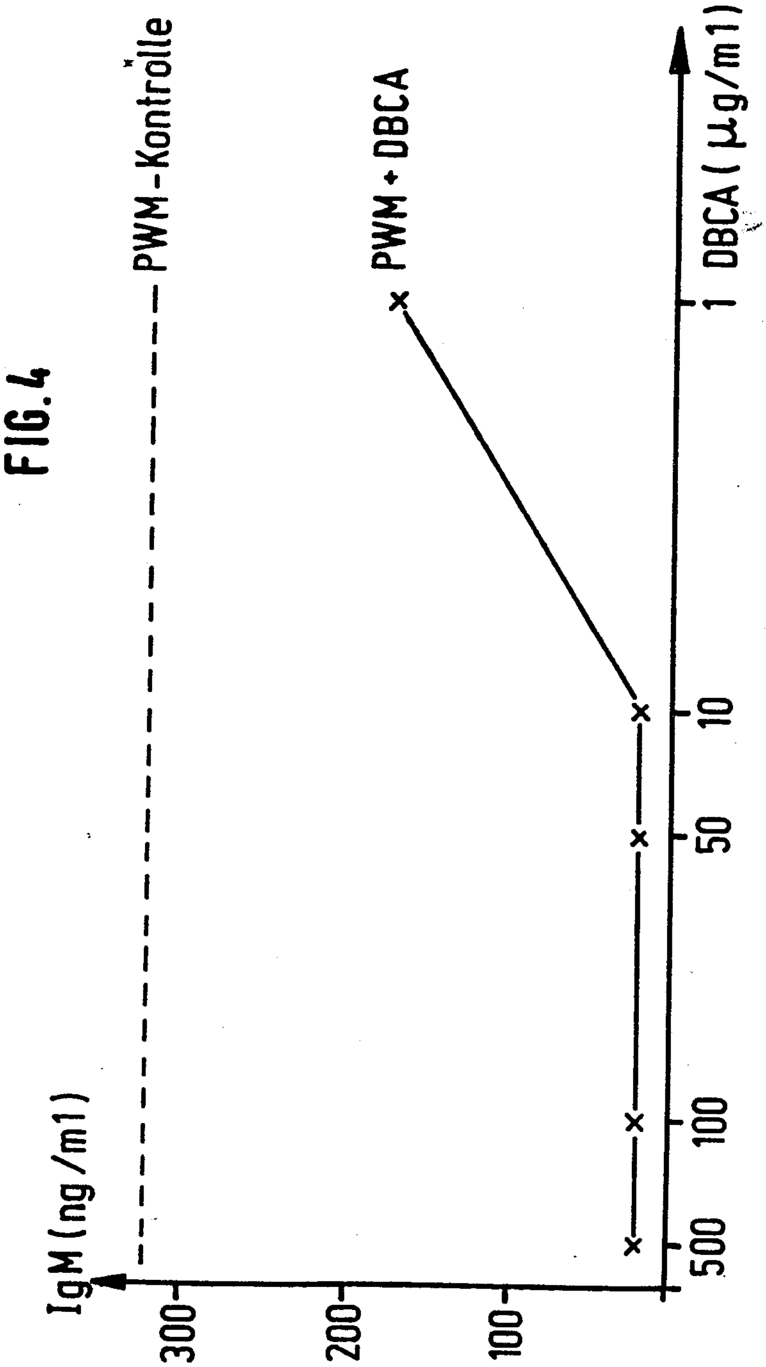
IgM (ng/ml)
300
200
100
PWM-Kontrolle
PWM + DBCA
500
100
50
10
1
DBCA (µg/ml)

0137193

FIG. 5